# EUROPÄISCHE PATENTSCHRIFT

(11) **EP 1 315 451 B1**
(45) Veröffentlichungstag und Bekanntmachung des Hinweises auf die Patenterteilung: **29.06.2005**
(21) Anmeldenummer: 01964924.3
(22) Anmeldetag: 28.08.2001
(51) Int. Cl.: A61B 5/18

(54) **VERFAHREN UND VORRICHTUNG ZUR DIAGNOSE DER FAHRTÜCHTIGKEIT EINES FAHRERS IN EINEM KRAFTFAHRZEUG**
METHOD AND DEVICE FOR DIAGNOSING IN A MOTOR VEHICLE A DRIVER'S FITNESS TO DRIVE
PROCEDE ET DISPOSITIF POUR LE DIAGNOSTIC DE L'APTITUDE A LA CONDUITE DU CONDUCTEUR D'UN VEHICULE AUTOMOBILE

(30) Priorität: 29.08.2000 DE 10042367
(43) Veröffentlichungstag der Anmeldung: 04.06.2003
(73) Patentinhaber: ROBERT BOSCH GMBH, 70442 Stuttgart (DE)
(72) Erfinder: KOENIG, Winfried, 76327 Pfinztal (DE)
(86) Internationale Anmeldenummer: PCT/DE2001/003232
(87) Internationale Veröffentlichungsnummer: WO 2002/017786

(56) Entgegenhaltungen:
- EP-A- 0 713 675
- WO-A-00/44580
- DE-A- 3 826 943
- DE-A- 19 643 593

## Beschreibung

### Stand der Technik

Die Erfindung betrifft ein Verfahren zur Diagnose der Fahrtüchtigkeit eines Fahrers in einem Kraftfahrzeug, bei dem aus vom Fahrer während der Fahrt im Fahrzeug abgeleiteten physiologischen Meßwerten Veränderungen des Fahrzustands ermittelt und, wenn die Veränderungen gravierend sind, eine Warnung ausgegeben oder Hilfsmaßnahmen eingeleitet werden, sowie eine Vorrichtung zur Durchführung dieses Verfahrens.

Es sind bereits Systeme bekannt, die den Zustand des Fahrers aufgrund von im Fahrzeug gemessenen physiologischen Parametern schätzen. Dazu gehören z.B. interaktive Systeme, die von einem Fahrer während der Fahrt bestimmte Eingabehandlungen fordern, anhand deren die Fahrtüchtigkeit des Fahrers bestimmt wird. Ferner gibt es Systeme, die die Augenlidschlagfrequenz über eine Videokamera mit nachgeschalteter Bildauswertung erfassen. Aus der erfaßten Lidschlagfrequenz lassen sich wiederum Rückschlüsse ziehen auf die Fahrtüchtigkeit eines Fahrers im Kraftfahrzeug.

Aus DE 196 43 593 A 1 ist eine Vorrichtung und ein Verfahren zum frühzeitigen Erkennen und Verhindern unzulässiger Konzentrationsschwächen und des Einschlafens beim Autofahren bekannt. Dabei werden Sensoren eingesetzt, die beim Fahrer während der Fahrt physiologische Daten wie die Augenlidschlagfrequenz, die Pulsrate im Blutdruck u. s. w. bestimmen. Dabei wird in einer Phase in der der Fahrer die volle Konzentration aufweist Sollwerte gewonnen und während späterer Phasen werden diese Sollwerte mit aktuellen Istwerten verglichen, um festzustellen, ob der Fahrer immer noch konzentriert ist. Aus EP 713 675 A2 ist eine Fahrtüchtigkeitsbeurteilungsvorrichtung bekannt, bei der nicht nur physiologische Daten berücksichtigt werden, sondern auch Straßenreisedaten, die beispielsweise von einem Navigationssystem abgeleitet werden, um in Abhängigkeit davon gegebenenfalls ein Alarm auszulösen.

Weiterhin gibt es im häuslichen Bereich Systeme, wie z.B. mit Ergometriefunktionen ausgestattete Trimmgeräte, die Aussagen über den Gesundheitszustand des Benutzers durch physiologische Meßwerte gestatten, wie z.B. Blutdruck, Pulsfrequenz, Hautimpedanz, etc.

Die Aussagekräftigkeit bisher bekannter bzw. in Entwicklung befindlicher Systeme zur Abschätzung der Fahrtüchtigkeit des Fahrers im Kraftfahrzeug ist allerdings erheblich dadurch eingeschränkt, daß nur Verfahren möglich sind, die keine festen Elektroden oder Kanülen am Körper des Fahrers verwenden, sondern mit Kontakten über unbedeckte Hautflächen auskommen müssen oder mit vollständig berührungslosen Messungen, wie die oben genannte Erfassung der Augenlidschlagfrequenz mittels einer Videokamera.

### Aufgabe und Vorteile der Erfindung

Es ist Aufgabe der Erfindung, die Sicherheit und Aussagekräftigkeit eines gattungsgemäßen Verfahrens zur Diagnose der Fahrtüchtigkeit eines Fahrers eines Kraftfahrzeugs zu steigern, um gegebenenfalls eine auf der Diagnose der Fahrtüchtigkeit beruhende Warnung an den Fahrer abzugeben und notfalls Hilfsmaßnahmen einzuleiten.

Gemäß einem wesentlichen Aspekt werden bei dem erfindungsgemäßen Verfahren die momentan während der Fahrt gemessenen physiologischen Meßwerte mit im häuslichen Bereich ermittelten stationären gesundheitsrelevanten Daten des Fahrers kombiniert und mit im Fahrzeug vorhandenen oder abgeleiteten, die Fahrerbelastung angebenden Daten, insbesondere über die momentane Verkehrssituation und den momentanen Fahrzustand des Fahrzeugs oder Veränderungen des Fahrerzustands von einem Expertensystem gewichtet und interpretiert.

Auf diese Weise können die im Fahrzeug aus dem Fahrzustand des Fahrzeugs und der momentanen Verkehrssituation gewonnenen Daten dazu verwendet werden, einen Schätzwert über die momentane Fahrerbelastung abzuleiten, mit dem dann die während der Fahrt im Fahrzeug gemessenen momentanen physiologischen Daten des Fahrers und die damit kombinierten, im häuslichen Bereich gewonnenen, gesundheitsrelevanten stationären Daten zu einer umfassenden Abschätzung der Fahrtüchtigkeit des Fahrers mit höherer Aussagekraft gewichtet und interpretiert werden.

Zur Erfassung physiologischer Werte des Fahrers im Fahrzeug bieten sich insbesondere Kreislaufwerte wie Pulsschlag und Blutdruck des Fahrers an. Derartige Kreislaufwerte können unschwer mit bereits bekannten Meßwertaufnehmern, z.B. in Form einer Armbanduhr, gewonnen und drahtlos an einen Empfänger im Fahrzeug übertragen werden.

Weiterhin bietet sich die Messung der Hautimpedanz des Fahrers im Fahrzeug an. Auch dieser physiologische Meßwert läßt sich mit bereits bekannten Meßwertaufnehmern, die an der Haut des Fahrers angebracht sind, gewinnen und drahtlos an einen Empfänger im Fahrzeug übertragen.

Erwähnt wurde auch bereits die mit einer Videokamera mit nachgeschalteter Bildverarbeitung mögliche Messung der Lidschlagparameter, wie z.B. Frequenz und Geschwindigkeit des Lidschlags des Fahrers, die eine deutliche Aussage über die Ermüdung des Fahrers bringen können.

Aus ohnehin im Fahrzeug vorhandenen oder ableitbaren Daten kann eine Schätzung der momentanen Belastung des Fahrers durch die Verkehrs- Umwelt- und Fahrsituation vorgenommen werden. So können z.B. Daten aus einem Zielführungssystem dienen. Sie geben Hinweise, in welcher statischen Verkehrssituation sich der Fahrer befindet, z.B. innerorts, außerorts, in komplexer Kreuzung, auf schmaler Gebirgsstraße. Hinweise über die dynamische Verkehrssituation erhält man aus Straßenzustandsdaten, Wetterdaten, Uhrzeit, durch die erfaßte Geschwindigkeit des eigenen Fahrzeugs, wie z.B. Beschleunigungswerte und -frequenz, und durch die Geschwindigkeit anderer Fahrzeuge, z.B. aufgrund von ACC-Signalen. Eine Schätzung der momentanen Verkehrsdichte erscheint aufgrund von ACC-Daten und mit Hilfe künftiger Videosensorik prinzipiell möglich.

Bei dem erfindungsgemäßen Verfahren wird nun die vorgenommene Schätzung der momentanen Fahrerbelastung durch ein Expertensystem in Verbindung gebracht mit der momentanen physiologischen Situation des Fahrers, wie sie die oben erwähnten mit Hilfe einer Fahrerzustandssensorik erfaßten physiologischen Werte liefern. Durch diese Verknüpfung kann festgestellt werden, wie sich die Belastung durch die Fahrzeugführung in den physiologischen Daten widerspiegelt. Dabei wird angenommen, daß andere Einflüsse, wie z.B. die Interaktion mit Beifahrern oder mit Gesprächspartnern über Mobiltelefon im Straßenverkehr einen deutlich geringeren Einfluß auf die Beanspruchung des Fahrers haben. Die Belastung durch die Fahrzeugführung dient gewissermaßen als "Ergometer", mit dem ein Patient, d.h. hier der Fahrer in eine bekannte Belastungssituation gebracht wird. Der Unterschied ist hier auch, daß die Fahrzeugführung eher eine geistig-seelische als eine körperliche Belastung darstellt. Aus der physiologischen Reaktion des Fahrerorganismus' auf diese Belastung können nun Aussagen über den gesundheitlichen Zustand des Fahrers gewonnen werden. So können z.B. extreme systolische Blutdruckwerte und hohe Pulsfrequenzen auftreten, und es läßt sich feststellen, ob diese mit schwierigen Verkehrssituationen korreliert sind oder bereits ohne derartige Belastungen auftreten. In letzterem Fall kann der Fahrer gewarnt werden und man kann ihm nahelegen, ärztliche Hilfe in Anspruch zu nehmen. Hinweise auf Übermüdung, Drogen, wie Blutalkoholgehalt, können in bekannter Weise aus Meßwerten der im Fahrzeug betriebenen Fahrerzustandssensorik gewonnen werden. Durch diese Gewichtung mit der aus der Verkehrssituation und dem Fahrzustand des Fahrzeugs geschätzten Fahrerbelastung können erheblich bessere Aussagen gemacht werden über die Relevanz dieser Hinweise.

Bei dem erfindungsgemäßen Verfahren wird, wie erwähnt, außerdem eine Kombination der durch die mobile Fahrerzustandssensorik im Fahrzeug gewonnenen momentanen physiologischen Daten mit stationär ermittelten gesundheitsrelevanten Daten des Fahrers vorgenommen. Derartige stationäre Gesundheitsdaten können z.B. durch Haushaltssysteme ermittelt werden, die in der Lage sind, aus biologischem Material der Nutzer, in diesem Fall des Fahrers, wie Urin, Stuhl und Schweiß etc., Information über dessen Gesundheitszustand zu gewinnen. Auch in häuslichen Gesundheitsmöbeln, wie in Fahrradergometern, Trimmgeräten, Blutzuckermeßgeräten, Körperwaagen etc., können gesundheitsrelevante Daten erhoben und gespeichert werden. Diese stationären Daten sind meist in statischen Situationen oder bei Muskelarbeit gewonnen und nicht überwiegend bei geistig-seelischer Belastung, wie es im Kraftfahrzeug auftritt. Die stationären Daten können nun in einem geeigneten Speicher-/Übertragungsmedium gespeichert und dieses von einem Eingabemittel im Fahrzeug gelesen oder die gespeicherte Information zu einem Empfänger im Fahrzeug gesendet werden. Dieses Speicher-/Übertragungsmedium kann z.B. eine Chipkarte und ein Mobilfunksystem un sein.

Das Expertensystem im Kraftfahrzeug bewertet die mit der mobilen Fahrzustandssensorik während der Fahrt gewonnenen momentanen physiologischen Daten mit den stationär gewonnenen gesundheitsrelevanten Daten des Fahrers und erhält damit ein umfassenderes Bild vom Gesundheitszustand des Fahrers, so daß bei gravierenden Abweichungen von Normwerten gegebenenfalls Hilfsmaßnahmen eingeleitet werden können.

Im Falle, daß keine stationären Meßwerte über den gesundheitlichen Zustand im häuslichen Bereich des Fahrers gewonnen werden können, werden von dem erfindungsgemäß vorgeschlagenen Diagnoseverfahren Standardwerte angenommen und diese Standardwerte mit eingegebenen biographischen Daten, wie Geschlecht, Alter und Gewicht des Fahrers präzisiert.

Bei einer Ausführungsform kann das Expertensystem als lernendes System eingerichtet sein, wobei am Anfang, bevor eine gültige Diagnose zur Fahrtüchtigkeit abgegeben wird, eine Lernphase mit mindestens einer Testfahrt des Fahrers in diesem Fahrzeug absolviert wird, die unterschiedliche Belastungsgrade und Verkehrssituationen umfaßt.

Eine zur Durchführung des Verfahrens eingerichtete Vorrichtung enthält das in einem Bordcomputer des Fahrzeugs implementierte Expertensystem, und der Bordcomputer steht über ein Fahrzeugbussystem in Verbindung mit der die physiologischen Meßwerte liefernden Fahrerzustandssensorik, einem die stationär ermittelten gesundheitsrelevanten und/oder biographischen Daten zuführenden Speicher-/Übertragungsmittel, mit Mitteln, die aus der momentanen Verkehrssituation und dem momentanen Fahrzustand des Fahrzeugs die Fahrerbelastung schätzen und an den Bordcomputer übertragen und auch mit Mitteln, die ein eine Überlastung oder eine nicht situationsgerechte Beanspruchung des Fahrers angebendes Signal dem Fahrer als ein Warnsignal mitteilen und/oder an die Umgebung aussenden. Zur Ermittlung der momentanen Verkehrssituation und Abschätzung der Fahrerbelastung kann ein ACC-System und ein Fahrzeugnavigationssystem verwendet werden. Zur Identifikation des Fahrers kann der Bordcomputer bzw. das darin residierende Expertensystem mit einer Fahrerzugangsberechtigungs-Kontrolleinheit in Verbindung stehen.

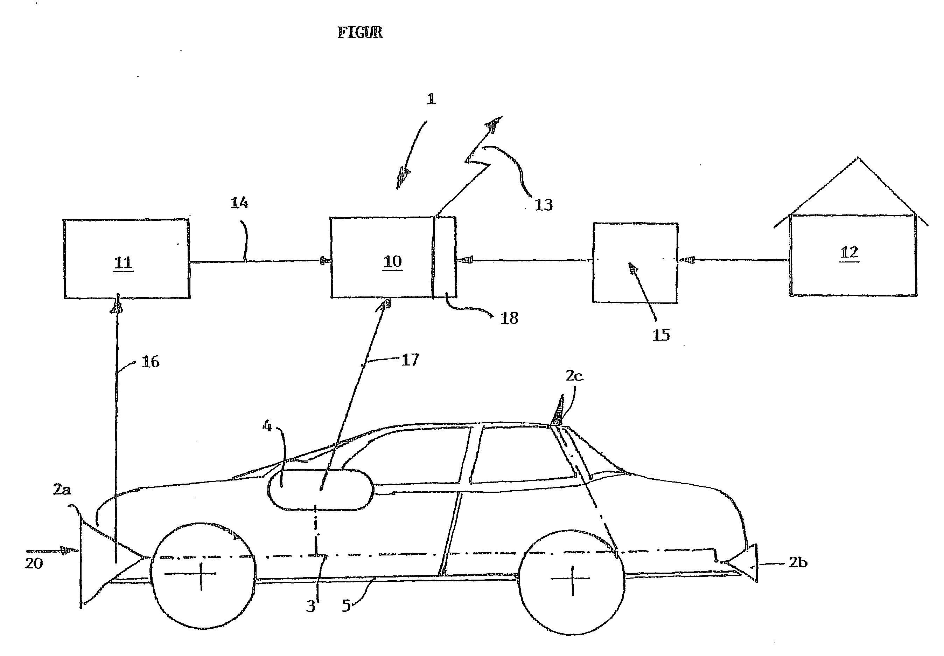

Die nachfolgende Beschreibung beschreibt anhand eines in der Figur dargestellten Funktionsblockdiagramms Schritte eines erfindungsgemäßen Fahrerdiagnoseverfahrens sowie Funktionen eines Ausführungsbeispiels einer zur Durchführung des Verfahrens eingerichteten Vorrichtung.

### Ausführungsbeispiel

Das in der Figur in Form von Funktionsblöcken dargestellte System 1 weist folgende Funktionseinheiten auf:
- ein in einem Bordcomputer implementiertes Expertensystem 10;
- ein ACC- und Navigationssystem 11 mit zugehörigen Sensoren 2a, 2b und einer Funkantenne 2c;
- Mittel 12 zur Erfassung der stationären (häuslichen) physiologischen Daten eines Fahrers;
- Mittel 15,18 zur Speicherung/Übertragung der stationär gewonnenen gesundheitsrelevanten Daten zum Bordcomputer;
- ein Fahrzeugbussystem 3 zur Verbindung verschiedener Einheiten im Fahrzeug, und
- eine im Fahrzeug 5 befindliche Fahrerzustandssensorik 4.

Nicht dargestellt ist eine optionell vorgesehene Fahrerzugangsberechtigungs-Kontrolleinheit.

Die dem ACC- und Navigationssystem 11 zugeordneten Sensoren 2a, 2b und die Funkantenne 2c dienen zur Erfassung der Umwelt/Verkehrssituation 20 z.B. über Radarwellen, Videobilderfassung und/oder Ultraschallwellen und zum Empfang von Wegeleitinformation über GSM-Mobilfunk. Im ACC- und Navigationssystem 11 werden von den entsprechenden Sensoren, 2a, 2b und der Antenne 2c erfaßte ACC-Information 20, wie Zahl, Geschwindigkeit und Kurs von Fremdfahrzeugen und Wegeleitinformation sowie eigener Kurs und Geschwindigkeit, Straßenklasse, innerorts/außerorts, Wetterdaten, Straßenzustand, Topographiedaten etc., ermittelt und daraus die Fahrerbelastung abgeschätzt, die in Form eines Signals 14 an das Expertensystem 10 im Bordcomputer übertragen wird. Ferner erhält das Expertensystem 10 von der mobilen Fahrerzustandssensorik 4 Signale 17, die die von der Fahrerzustandssensorik 4 erfaßten physiologischen Daten des Fahrers .im Fahrzeug angeben. Die im stationären, häuslichen System 12 ermittelten physiologischen und die biographischen Daten werden über das Speicher/-Übertragungsmedium 15, d.h. eine Chipkarte, durch ein Eingabe-/Lesemedium 18 dem Expertensystem 10 eingegeben. Darin werden die so eingegebenen stationären Gesundheitsdaten mit den von der mobilen Fahrerzustandssensorik 4 während der Fahrt erfaßten momentanen physiologischen Daten 17 des Fahrers verknüpft und mit dem Schätzwert 14 über die Fahrerbelastung gewichtet und interpretiert.

Das Expertensystem 10 gewichtet die physiologischen und biographischen Daten mit der geschätzten Fahrerbelastung 14 und erzeugt, wenn die Veränderungen im Fahrerzustand gravierend sind, eine Warnung für den Fahrer und/oder ein z.B. über GSM-Funk nach draußen ausgebbares Signal 13, das die Überlastung oder nicht situationsadäquate Beanspruchung des Fahrers angibt, so daß Hilfsmaßnahmen eingeleitet werden können. Der Bordcomputer bzw. das Expertensystem kann in einer Ausführungsform mit einer Fahrerzugangsberechtigungs-Kontrolleinheit in Verbindung stehen, die die Identifikation des Fahrers ausführt und dem Expertensystem zuleitet.

Kern des erfindungsgemäßen Verfahrens und der zur Durchführung des Verfahrens eingerichteten Vorrichtung ist somit die vom Expertensystem vorgenommene Zusammenführung der durch die Fahrerzustandssensorik 4 während der Fahrt gewonnenen physiologischen Daten 17 mit den im häuslichen Bereich gewonnen stationären, über das Speicher-/Übertragungsmedium 15 zugeführten gesundheitrelevanten Daten und die Gewichtung dieser kombinierten Daten durch einen die Fahrerbelastung angegebenen Schätzwert 14, der aus den vom bordeigenen ACC- und Navigationssystem 12 gewonnenen Verkehrssituations- und Wegeleitinformationen als unabhängige Variable abgeschätzt und dem Expertensystem 10 zugeführt wird. Aus der gewichteten Veränderung des Fahrerzustands entscheidet das Expertensystem 10, ob diese Veränderung so gravierend ist, daß die Fahrtüchtigkeit in Frage gestellt ist, ob dem Fahrer ein Warnsignal ausgegeben werden muß oder ob gegebenenfalls Hilfsmaßnahmen einzuleiten sind.

In dem oben beschriebenen Ausführungsbeispiel residiert das Expertensystem 10 in einem Bordcomputer eines Fahrzeugs 5. Das erfindungsgemäße Verfahren ist jedoch nicht auf dieses derzeit bevorzugte Ausführungsbeispiel beschränkt, sondern auch verwendbar, wenn das Expertensystem 10 außerhalb des Fahrzeugs 3 an einem zentralen Ort residiert, dem dann die durch die mobile Fahrerzustandssensorik ermittelten physiologischen Daten, die stationären physiologischen und biographischen Daten sowie die vom ACC-System und Navigationssystem 12 ermittelten Informationen über die Verkehrssituation die Wegeleitinformationen zugeführt werden.

Die Erfindung ist oben für einen Anwendungsfall zur Ermittlung der Fahrtüchtigkeit des Fahrers eines Kraftfahrzeugs beschrieben worden. Den einschlägigen Fachleuten ist es jedoch unmittelbar deutlich, daß die Erfindung auch zur Fahrtüchtigkeitsdiagnose bei Fahrern (Piloten) anderer Fahrzeuge und Verkehrsmittel, wie z.B. Schiffen, Flugzeugen, Schienenfahrzeugen anwendbar ist. Der in der obigen Beschreibung und den Ansprüchen verwendete Begriff Kraftfahrzeug oder Fahrzeug ist demnach weit zu fassen.

## Patentansprüche

1. Verfahren zur Diagnose der Fahrtüchtigkeit eines Fahrers eines Kraftfahrzeugs, bei dem aus vom Fahrer während der Fahrt im Fahrzeug abgeleiteten physiologischen Messwerten Veränderungen des Fahrerzustandes ermittelt und bewertet und, wenn die Veränderungen gravierend sind, eine Warnung ausgegeben oder Hilfsmaßnahmen eingeleitet werden, **dadurch gekennzeichnet, dass** die während der Fahrt im Fahrzeug erfassten physiologischen Messwerte von einem Expertensystem mit im häuslichen Bereich stationär ermittelten gesundheitsrelevanten Daten des Fahrers kombiniert werden, dass die Daten über ein Speichermedium eingeladen oder über Mobilfunk empfangen werden, und dass vom Expertensystem Veränderungen des Fahrerzustandes mit einer die Fahrerbelastung angebenden Größe, die aus im Fahrzeug vorhandenen oder abgeleiteten Daten abgeschätzt wird zur Erkennung, ob der Fahrer fahrtüchtig ist oder nicht, gewichtet und interpretiert werden.

2. Verfahren nach Anspruch 1, **dadurch gekennzeichnet, dass** als physiologische Messwerte Kreislaufmesswerte des Fahrers im Fahrzeug erfasst werden.

3. Verfahren nach Anspruch 1 oder 2, **dadurch gekennzeichnet, dass** als physiologischer Messwert die Impedanz der Haut des Fahrers im Fahrzeugs erfasst wird.

4. Verfahren nach einem der vorhergehenden Ansprüche, **dadurch gekennzeichnet, dass** als physiologischer Messwert ein Elektrokardiogramm des Fahrers im Fahrzeug gemessen wird.

5. Verfahren nach einem der vorhergehenden Ansprüche, **dadurch gekennzeichnet, dass** als physiologischer Messwert die Lidschlagfrequenz des Fahrers im Fahrzeug erfasst wird.

6. Verfahren nach einem der vorhergehenden Ansprüche, **dadurch gekennzeichnet, dass** als gesundheitsrelevante Daten im häuslichen Bereich des Fahrers stationär gemessene physiologische Daten, wie z.B. an Ergometergeräten gemessene ergometrische Daten und biographische Daten, wie Alter, Gewicht und Geschlecht, verwendet werden.

7. Verfahren nach einem der vorhergehenden Ansprüche, **dadurch gekennzeichnet, dass** als gesundheitsrelevante stationäre Daten im häuslichen Bereich gewonnene Blutanalysewerte verwendet werden.

8. Verfahren nach einem der vorhergehenden Ansprüche, **dadurch gekennzeichnet, dass** die momentane Verkehrssituation aus Anzahl, Geschwindigkeit und Kurs von Fremdfahrzeugen und relevanter Wegeleitinformation für das Fahrzeug geschätzt, mit momentanen Fahrzustandsdaten des betreffenden Kraftfahrzeugs kombiniert und daraus die Fahrerbelastung berechnet wird.

9. Verfahren nach einem der vorhergehenden Ansprüche, **dadurch gekennzeichnet, dass** als Expertensystem ein lernendes System verwendet wird und dass am Anfang, bevor eine gültige Diagnose der Fahrtüchtigkeit abgegeben wird, eine Lernphase mit mindestens einer unterschiedliche Belastungsgrade und Verkehrssituationen umfassenden Testfahrt des Fahrers mit diesem Fahrzeug absolviert wird.

10. Verwendung einer Vorrichtung zur Durchführung des Verfahrens nach Anspruch 1, **dadurch gekennzeichnet, dass** das Expertensystem (10) in einem Bordcomputer des Kraftfahrzeugs (5) implementiert ist, der durch einen Fahrzeugbus (3) in Verbindung steht mit einer die physiologischen Messwerte liefernden fahrzeugbasierten Fahrerzustandssensorik (4), einem die stationär ermittelten gesundheitsrelevanten Daten und/oder die biographischen Daten zuführenden Speicher-/Übertragungsmittel (15, 18), mit Mitteln (11) zur Ermittlung und Übertragung der momentanen Verkehrssituation (20) und der momentanen Fahrzustandsdaten des Kraftfahrzeugs (5) an den Bordcomputer sowie mit Sendemitteln (13) zur Übertragung eines eine Überlastung oder nicht situationsgerechte Beanspruchung des Fahrers angebenden Signals an die Umgebung.

## Claims

1. Method for diagnosis of whether a driver is fit to drive a vehicle, in which changes in the driver state are determined and assessed from physiological measured values derived from the driver while the vehicle is being driven, and if the changes are serious, a warning is emitted or remedial measures are initiated, **characterized in that** the physiological measured values which are recorded while the vehicle is being driven are combined by an expert system with health-relevant driver data determined in the steady state in the domestic environment, **in that** the data is loaded via a storage media or is received by mobile radio, and **in that** changes in the driver state are weighted and interpreted by the expert system by means of a variable which indicates the driver load and is estimated from data which exists or is derived in the vehicle in order to identify whether or not the driver is fit to drive.

2. Method according to Claim 1, **characterized in that** driver circulation measured values are recorded as physiological measured values in the vehicle.

3. Method according to Claim 1 or 2, **characterized in that** the impedance of the driver's skin in the vehicle is recorded as a physiological measured value.

4. Method according to one of the preceding claims, **characterized in that** an electrocardiogram of the driver in the vehicle is measured as a physiological measured value.

5. Method according to one of the preceding claims, **characterized in that** the driver's eyelid blinking frequency in the vehicle is recorded as a physiological measured value.

6. Method according to one of the preceding claims, **characterized in that** physiological data which is measured in the steady state in the driver's domestic environment, such as ergometric data measured on ergometer appliances and biographical data such as age, weight and gender, are used as health-relevant data.

7. Method according to one of the preceding claims, **characterized in that** blood analysis values obtained in the domestic environment are used as health-relevant steady state data.

8. Method according to one of the preceding claims, **characterized in that** the instantaneous traffic situation is estimated from the number, speed and path of other vehicles and relevant routing information for the vehicle, is combined with instantaneous driving state data for the relevant motor vehicle, and the driver load is calculated from this.

9. Method according to one of the preceding claims, **characterized in that** a learning system is used as the expert system, and **in that** at the start, before a final diagnosis of the fitness to drive is output, a learning phase is carried out by means of at least one test drive, which comprises different load levels and traffic situations, by the driver, with this vehicle.

10. Use of an apparatus for carrying out the method according to Claim 1, **characterized in that** the expert system (10) is implemented in an on-board computer in the motor vehicle (5), which is connected by means of a vehicle bus (3) to a vehicle-based driver state sensor system (4) which produces the physiological measured values, to storage/transmission means (15, 18) which supply the biographical data and/or the health-relevant data determined in the steady state, to means (11) for determination and transmission of the instantaneous traffic situation (20) and the instantaneous driving state data for the motor vehicle (5) to the on-board computer, and to transmission means (13) for transmission to the environment of a signal which indicates overloading or that the driver is not responding appropriately to the situation.

## Revendications

1. Procédé pour le diagnostic de l'aptitude à la conduite du conducteur d'un véhicule automobile, selon lequel on détecte et on évalue des variations de l'état du conducteur d'après des valeurs physiologiques mesurées sur le conducteur pendant la conduite dans le véhicule et, lorsque les variations sont importantes, on émet une alarme ou on enclenche des mesures auxiliaires,
**caractérisé en ce que**
à l'aide d'un système expert, on combine, les valeurs de mesure physiologiques détectées pendant la conduite dans le véhicule avec des données relatives à la santé du conducteur déterminées au repos dans un contexte familial, on charge les données à l'aide d'un support de mémoire ou on les reçoit par téléphonie mobile, et on pondère et on interprète, à l'aide du système expert, les variations de l'état du conducteur avec une grandeur indiquant la charge du conducteur, qui est évaluée à partir des données existantes ou mesurées dans le véhicule, pour reconnaître si le conducteur est ou non apte à la conduite.

2. Procédé selon la revendication 1,
**caractérisé en ce que**
comme valeurs de mesure physiologiques on détecte des valeurs de mesure de la circulation sanguine du conducteur dans le véhicule.

3. Procédé selon la revendication 1 ou 2,
**caractérisé en ce que**
comme valeur de mesure physiologique on détecte l'impédance de la peau du conducteur dans le véhicule.

4. Procédé selon l'une quelconque des revendications précédentes,
**caractérisé en ce que**
comme valeur de mesure physiologique on mesure un électrocardiogramme du conducteur dans le véhicule.

5. Procédé selon l'une quelconque des revendications précédentes,
**caractérisé en ce que**
comme valeur de mesure physiologique on détecte la fréquence de battement des paupières du conducteur dans le véhicule.

6. Procédé selon l'une quelconque des revendications précédentes,
**caractérisé en ce que**
comme données relatives à la santé on utilise des données physiologiques mesurées au repos dans le cadre familial du conducteur, comme par exemple des données ergométriques mesurées sur des appareils ergométriques et des données biographiques comme l'âge, le poids et le sexe.

7. Procédé selon l'une quelconque des revendications précédentes,
**caractérisé en ce que**
comme données relatives à la santé mesurées au repos on utilise des valeurs de l'analyse sanguine obtenues dans le cadre familial.

8. Procédé selon l'une quelconque des revendications précédentes,
**caractérisé en ce qu'**
on évalue la situation instantanée du trafic, à partir du nombre, de la vitesse et de la trajectoire des autres véhicules, et d'informations de radioguidage relatives au véhicule, on la combine à des données instantanées de l'état de marche du véhicule automobile concerné, et on à partir de cela on calcule la charge du conducteur.

9. Procédé selon l'une quelconque des revendications précédentes,
**caractérisé en ce que**
comme système expert on utilise un système adaptatif, et au début, avant d'émettre un diagnostic valable de l'aptitude à la conduite, on effectue une phase d'adaptation comportant au moins une conduite d'essai du conducteur avec ce véhicule, dans des conditions comprenant différents degrés de charge et différentes situations de trafic.

10. Utilisation d'un dispositif pour la mise en oeuvre du procédé selon la revendication 1,
**caractérisée en ce que**
le système expert (10) est mis en oeuvre dans un ordinateur de bord du véhicule automobile (5), qui est en communication par un bus de véhicule (3) avec une unité de détection de l'état du conducteur (4) basée sur le véhicule et fournissant les valeurs de mesure physiologiques, avec un moyen de stockage/transmission (15, 18) fournissant les données relatives à la santé déterminées au repos et/ou les données biographiques, avec des moyens (11) pour déterminer et transmettre la situation instantanée du trafic (20) et les données instantanées de l'état de marche du véhicule (5) à l'ordinateur de bord ainsi qu'avec des moyens d'émission (13) pour transmettre à l'entourage un signal indiquant une surcharge ou une sollicitation du conducteur non adaptée à la situation.
